Europäisches Patentamt

European Patent Office

Office européen des brevets

(19)

(11) Numéro de publication: **0 274 961 B1**

# (12) FASCICULE DE BREVET EUROPEEN

(45) Date de publication de fascicule du brevet: **25.03.92**  (51) Int. Cl.5: **B01J 13/02**, A61K 9/50

(21) Numéro de dépôt: **87402998.6**

(22) Date de dépôt: **28.12.87**

(54) **Procédé de préparation de systèmes colloidaux dispersibles d'une substance, sous forme de nanocapsules.**

(30) Priorité: **31.12.86 FR 8618444**

(43) Date de publication de la demande:
**20.07.88 Bulletin 88/29**

(45) Mention de la délivrance du brevet:
**25.03.92 Bulletin 92/13**

(84) Etats contractants désignés:
**AT BE CH DE ES FR GB GR IT LI LU NL SE**

(56) Documents cités:
**FR-A- 1 571 090**
**GB-A- 1 516 348**

**PHARM. ACTA HELV., vol. 53, no. 2, 1978, pages 33-39; J. KREUTER: "Nanoparticles and nanocapsules- new dosage forms in the nanometer size range"**

(73) Titulaire: **CENTRE NATIONAL DE LA RECHER-CHE SCIENTIFIOUE (CNRS)**
**15, Ouai Anatole France**
**F-75007 Paris(FR)**

(72) Inventeur: **Fessi, Hatem**
**9 rue Friant**
**F-75014 Paris(FR)**
Inventeur: **Puisieux, Francis**
**66 rue de Strasbourg**
**F-94700 Maisons Alfort(FR)**
Inventeur: **Devissaguet, Jean-Philippe**
**14 Bd d'Inkermann**
**F-92200 Neuilly S/Seine(FR)**

(74) Mandataire: **Varady, Peter et al**
**Cabinet Lavoix 2, Place d'Estienne d'Orves**
**F-75441 Paris Cedex 09(FR)**

Description

La présente invention a pour objet un nouveau procédé de préparation de systèmes colloïdaux dispersibles d'une substance sous forme de particules sphériques de type vésiculaire et de taille inférieure à 500 nm (nanocapsules).

Des systèmes nano-vésiculaires, comprenant des nanocapsules ayant une taille de 50 à 500 nm et constituées d'un noyau central solide ou liquide enveloppé par une membrane continue insoluble dans l'eau, ont déjà été décrits dans la littérature. Les constituants de la membrane peuvent être des macromolécules synthétiques (polymères) ou naturelles (protéines), ou des lipides (liposomes).

Des particules de polymères submicroscopiques d'un diamètre inférieur à 500 nm sont connues notamment d'après les brevets BE-A-808 034, BE-A-839 748, BE-A-869 107, FR-A-2 504 408 et FR-A-2 515 960. Selon BE-A-808 034 et -839 748, les particules submicroscopiques sont formées par polymérisation micellaire d'un monomère tel qu'un dérivé de l'acide acrylique. De même, BE-A-869 107, FR-A-2 504 408 et FR-A-2 515 960 décrivent la préparation de nanoparticules et de nanocapsules biodégradables obtenues par polymérisation d'un cyanoacrylate d'alkyle et contenant une substance biologiquement active. Les méthodes font appel à une polymérisation en solution et sont donc limitées à l'utilisation d'un nombre limité de polymères pouvant être obtenus notamment par vinyl-addition et ne peuvent convenir à des polymères naturels ou hémisynthétiques. De plus il est difficile de maîtriser le poids moléculaire du polymère constitutif des nanoparticules et il est nécessaire, notamment en vue d'usage biologique, d'éliminer les monomères et oligopolymères résiduels, le cas échéant les réactifs de polymérisation (initiateur et catalyseur) en excès, ainsi que les surfactifs s'ils sont utilisés à forte concentration ou ne sont pas biocompatibles. Or la purification (ultracentrifugation, dialyse) s'avère souvent lourde, car la filtration des nanoparticules et des nanocapsules, vu leur taille, n'est pas toujours possible.

On a aussi décrit la préparation de nanocapsules protéiques, notamment de gélatine. Ainsi, Krause et col. (Pharm. Research, 239 (1985)), obtiennent des nanocapsules de gélatine dont le contenu est lipophile (chloroforme), à partir d'une émulsion de chloroforme dans une phase continue aqueuse (solution d'albumine). Cette émulsion est ensuite soumise à une désolvatation par le sulfate de sodium. Les nanocapsules sont ensuite durcies par le glutaraldéhyde, dont l'excès doit être détruit par du métabisulfite de sodium qui doit être éliminé. De plus, la stabilité du chloroforme dans la nanocapsule semble limitée par son évaporation : les nanocapsules peuvent se vider du solvant, mais conservent le produit lipophile initialement en solution dans le chloroforme.

Les lipsomes unilamellaires peuvent être considérés comme des nanovésicules. Leur préparation s'effectue par sonication intense d'une dispersion aqueuse de phospholipides, qui conduit à une taille particulaire de 20 à 100 nm, mais présente l'inconvénient des risques d'oxydation et autres altérations liées à la sonication. Batzri et col. (Biochim. Biophys. Acta, 298, 1015 (1973)) proposent une méthode sans sonication qui procède par injection (seringue Hamilton) d'une solution de lécithine d'oeuf dans une phase aqueuse et conduit à des liposomes unilamellaires d'environ 25 nm. Il s'agit cependant d'une méthode de laboratoire qui n'est pas extensible à d'autres types de produits.

Par ailleurs, le document FR-A-1 571 090 décrit un procédé de préparation de microcapsules de matières huileuses avec paroi polymère utilisant une méthode par dilution.

Finalement, AU-A-495 261 décrit un procédé de préparation de nanoparticules de 10 à 1000 nm à base d'une matière de macromolécules naturelles réticulées, utilisant une méthode de désolvatation/fixation.

Toutes les méthodes décrites ci-dessus ne sont applicables qu'à certaines catégories de molécules et font intervenir des opérations coûteuses (ultracentrifugation, sonication, etc) ou difficilement contrôlables (polymérisation), tout en ne permettant pas d'obtenir une homogénéité satisfaisante de la taille des particules ou même des particlues suffisamment petites (inférieures à 500 nm) pour assurer leur stabilité à long terme sous forme d une suspension colloïdale.

L'invention propose un nouveau procédé de préparation de nanocapsules, ne présentant pas les désavantages précédents et utilisable aussi bien pour des substances polymèriques synthétiques et naturelles que pour diverses substances organiques (médicaments, lipides, etc) ou minérales (sels, pigments, etc), ainsi que leurs mélanges.

L'invention a pour objet un procédé de préparation de systèmes colloïdaux dispersibles, sous forme de particules sphériques de type vésiculaire et de taille inférieure à 500 nm (nanocapsules), dont la paroi est constituée par une substance polymère A et dont le noyau est constitué par une substance B, caractérisé en ce que :

(1) on prépare une phase liquide constituée essentiellement par une solution de la substance polymère A dans un solvant ou dans un mélange de solvants contenant la substance B en solution ou en dispersion,

(2) on prépare une seconde phase liquide constituée essentiellement par un non-solvant ou un mélange de non-solvants des substances A et B, et additionnée d'un ou de plusieurs surfactifs, le solvant ou le mélange de solvants de la première phase étant miscible en toutes proportions au non-solvant ou au mélange de non-solvants de la seconde phase,

(3) on ajoute, sous agitation modérée la première phase à la seconde phase, de manière à obtenir une suspension colloïdale de nanocapsules,

(4) si l'on désire, on élimine tout ou partie du solvant ou du mélange de solvants et du non-solvant ou du mélange de non-solvants, de manière à obtenir une suspension colloïdale de concentration voulue en nanocapsules ou à obtenir, après addition de substances stabilisantes, une poudre de nanocapsules.

Dans l'étape (3), les nanocapsules se forment pratiquement instantanément. La solution devient blanche, laiteuse et présente l'effet Tyndall caractéristique des suspensions colloïdales. Dans cette étape, on ajoute de préférence la phase liquide obtenue dans l'étape (1) à la phase liquide obtenue dans l'étape (2), notamment si celle-ci est aqueuse.

La "substance A" est un polymère, aussi bien un polymère synthétique, par exemple l'acide poly (d,l) lactique (PLA), etc, un polymère hémisynthétique comme par exemple l'acétate butyrate de cellulose, l'éthylcellulose, le phtalate d'hydroxyméthylpropylcellulose (HPMCP), etc, qu'un polymère naturel, par exemple la gélatine, la gomme arabique. De nombreux autres polymères peuvent être utilisés, par exemple : l'acéto-phtalate de polyvinyle, l'acéto-phtalate de cellulose; des dérivés maléiques (par ex. "Gantrez®"); les copolymères d'acide acrylique et d'acrylates et les polymères acryliques (par ex. Eudragit®); l'acide polylactique d ou l, et d,l); les copolymères d'acide lactique et d'acide glycolique, polypeptides, glycolides (dérivés de propiolactone, butyrolactone, pivalolactone, ε -caprolactone); les polymères obtenus à partir d'esters cycliques des acides hydroxybutyrique, hydroxyisobutyrique, hydroxyméthylvalérique, pnényl-lactique, hydroxyéthylbutyrique; le poly béta malate de benzyle; les copolymères de l'acide malique et du malate de benzyle; un copolymère polyvinylpyrrolidone-acétate de vinyle réticulé, les polycyanoacrylates d'alkyle; les poly (éthylène-vinylacétate); les polymères hydrosolubles (gélatine, gomme arabique, méthylcellulose, etc); les oligomères (styrène allyl alcool).

La "substance B" que l'on désire encapsuler dans la substance A peut être pratiquement n'importe quelle substance soluble ou dispersible dans un solvant donné.

La "substance B" peut être une huile végétale ou minérale, ou toute substance huileuse, par exemple l'huile d'olive, le benzoate de benzyle, le myristate d'isopropyle, des glycérides d'acide gras (p. ex un Miglyol®), l'huile de bouquet.

La "substance B" peut aussi être une substance biologiquement active, notamment un principe actif médicamenteux ou un précurseur de principe actif médicamenteux, ou encore un produit de contraste ou un réactif biologique.

La "substance B" peut aussi être un pigment, une encre, un lubrifiant, un agent de traitement de surface.

Il est évident que le procédé selon l'invention peut s'appliquer aussi bien pour une substance B que pour plusieurs, par exemple une huile et une substance biologiquement active en solution dans cette dernière.

Le "solvant" ou le mélange de solvants utilisé est un liquide capable de dissoudre la substance A et éventuellement la substance B (par exemple le polymère et éventuellement la substance biologiquement active). Par ailleurs le solvant doit être miscible en toutes proportions avec le non-solvant de la substance utilisé dans la préparation. Ainsi, dans la plupart des cas, le solvant sera organique de sorte que la phase liquide (1) constituera la phase organique tandis que la phase liquide (2) pourra constituer la phase aqueuse, mais il est possible d'utiliser soit deux phases organiques soit deux phases aqueuses dans la mesure où les conditions de solubilité, d'insolubilité et de miscibilité sont remplies. D'un autre côté, le solvant devra être suffisamment volatil pour pouvoir éventuellement être éliminé. Par exemple, dans le cas où la substance est un polymère, le solvant peut être choisi parmi un alcool inférieur (méthanol, éthanol, isopropanol), une cétone inférieure (acétone, méthyl-éthyl-cétone), un hydrocarbure léger ou un mé-lange d'hydrocarbures légers (hexane, éther de pétrole) un hydrocarbure léger chloré (chloroforme, chlorure de méthylène, trichloréthylène), ou d'autres solvants légers usuels tels que l'acétonitrile, le dioxanne.

Le "non-solvant", ou le mélange de non-solvants des substances A et B est un liquide ne dissolvant pas ces substances tout en étant miscible en toutes proportions au solvant utilisé. Ainsi, par exemple, lorsque la subS_tance A est un polymère tel que P.L.A., le solvant peut être l'acétone et le non-solvant peut être l'éthanol ou l'eau distillée; si la substance A est par exemple un polymère acrylique tel que Eudragit L100$^R$, le solvant peut être une phase aqueuse alcaline et le non-solvant peut être une phase aqueuse acide.

Le ou les surfactifs (ou émulsifiants) ajoutés à la phase liquide (2) peuvent être naturels (lécithines), ou synthétiques anioniques (par exemple laurylsulfate de sodium), cationiques (par exemple ammonium quaternaire) ou non ioniques (par exemple monoesters de sorbitanne polyoxyéthylénés ou non, éthers d'alcools gras et de polyoxyéthylène-glycols, polyoxyéthylène-polypropylène glycol, etc). Toutefois, il est possible d'ajouter un ou plusieurs surfactifs du même type que ci-dessus à la phase liquide (1) notamment pour améliorer la stabilité de la suspension.

Dans l'étape (4) la totalité des solvants et des non-solvants peut être éliminée, par exemple par lyophilisation. On peut ainsi obtenir des nanocapsules lyophilisées dont la conservation est de longue durée.

La proportion en surfactifs dans la suspension colloïdale obtenue dans l'étape (3) lorsqu'ils sont présents, peut aller notamment de 0,1% à 10% en poids, et de préférence entre 0,2 et 2% en poids.

La concentration du polymère A dans le solvant ou le mélange de solvants peut être comprise entre 0,1 et 10%, de préférence entre 0,2 et 2% en poids.

Le rapport de volumes des solvants et des non-solvants doit être tel qu'il puisse permettre la précipitation du polymère. Lorsque ce rapport augmente, la taille des nanocapsules diminue.

L'agitation modérée de la préparation dans l'étape (3) est liée à la quantité de produits mise en oeuvre. Elle n' est pas nécessaire pour de faibles quantités.

L'influence de la température et du pH sont limitées dans le procédé selon l'invention, de sorte qu'il n'est généralement pas nécessaire d opérer dans des conditions particulières. Toutefois, lorsqu'on utilise deux phases aqueuses (1) et (2), leurs pH respectifs devront être différents, pour remplir respectivement les conditions de solvant et de non-solvant.

Par ailleurs, la présence d'électrolyte (par exemple le chlorure de sodium) ne semble pas avoir d'influence sur l'obtention des nanocapsules. Ainsi après formation des nanocapsules dans l'étape (3) une concentration de 25 mg/ml de chlorure de sodium n'entraîne pas de coalescence ou de précipitation des nanocapsules formées.

Les nanocapsules obtenues selon l'invention sont autoclavables si les propriétés physiques de la substance le permettent.

Le procédé d'obtention de nanocapsules selon l'invention apporte les avantages suivants par rapport aux procédés connus :

- obtention de nanocapsules inférieures à 500 nm et notamment d'environ 150 à 450 nm par une méthode simple ne nécessitant pas un grand apport d'énergie;
- la substance A formant la paroi étant un polymère, les nanocapsules ne sont plus obtenues par polymérisation d'un monomère mais par "nanoprécipitation" d'un polymère bien défini;
- utilisation de polymères naturels aussi bien que synthétiques dont l'innocuité est reconnue et l'application dans le domaine de la santé est très ancienne;
- utilisation de polymères qui, selon leur nature, peuvent être biocompatibles;
- possibilité d'utilisation de polymères qui peuvent se dissoudre à partir d'un certain pH dans l'organisme, d'où l'assurance d'une non-concentration dans l'organisme de particules de polymères;
- possibilité d'utilisation de polymères qui, selon leur nature peuvent être biorésorbables, les produits de dégradation étant d'une totale inocuité;
- obention de capsules sphériques avec une faible dispersion des tailles et un rapport contenu/contenant (par exemple un rapport principe actif/polymère très élevé.

Les exemples suivants illustrent l'invention. Les nanocapsules obtenues sont visibles au microscope électronique à transmission (x 25000-150000) et se présentent, après coloration négative avec l'acide phosphotungstique, sous la forme de particules non contrastées sensiblement rondes.

Example 1: Préparation de nanocapsules de polymère contenant un liquide organique

D'une part, 125 mg, de copolymère de chlorure de vinyle et d'acétate de vinyle (Rhodopas® AX 85-15) et 0,5 ml de benzoate de benzyle sont dissous dans 25 ml d'acétone.

D'autre part, 125 mg de polymère mixte d'oxyde d'éthylène et de propylène glycol (Pluronic F68® ou Poloxamer 188®), agent de surface non ionique, sont dissous dans 50 ml d'eau purifiée.

On verse, sous agitation magnétique modérée (environ 100 r.p.m.), la phase acétonique dans la phase aqueuse. Celle-ci devient immédiatement blanc laiteux avec des reflets bleutés par formation des nanocapsules dont la paroi est constituée par le copolymère vinylique et dont le noyau est constitué par le benzoate de benzyle.

L'acétone est éliminée sous pression réduite (vide de la trompe à eau) et la suspension est concentrée, par élimination de l'eau dans les mêmes conditions, jusqu'au volume désiré, par exemple 10 ml.

La suspension concentrée est filtrée sur verre fritté (pores de 9 - 15 µm) et la taille des nanocapsules mesurée dans un diffractomètre à rayon laser (Nanosizer® de la firme Coultronics) est de 410 nm avec un indice de dispersion de 2.

Example 2: Préparation de nanocapsules de polymères contenant de l'indométacine (principe actif lipophile)

a) D'une part, 125 mg de polyisobutylcyanoacrylate, 0,5 ml de benzoate de benzyle, et 15 mg d'indométacine, sont dissous dans 25 ml d'acétone.

D'autre part, 125 mg de polymère mixte d'oxyde d'éthylène et de propylèneglycol (Pluronic F68®) sont dissous dans 50 ml d'eau purifiée.

On procède alors comme indiqué dans l'exemple 1 et on obtient une suspension de nanocapsules dont la taille est de 240 nm avec un indice de dispersion de 2. Après ultracentrifugation, le dosage de l'indométacine dans la phase aqueuse dispersante mon-tre que les nanocapsules incluent 98% du principe actif.

b) Essai pharmacologique :

Administrée par voie orale chez le rat à jeun (5 mg/kg d'indométacine) la suspension de nanocapsules conduit à une absorption digestive de l'indométacine plus rapide et plus complète que celle observée après administration de la même dose d'indométacine en solution. Après administration répétée chez le rat à jeun (5mg/kg d'indométacine, 3 jours de suite), la suspension de nanocapsules conduit à une meilleure tolérance digestive, objectivée par le nombre des ulcérations et hémorragies, que celle observée après administration de la même dose d'indométacine en solution.

Adminsitrée par voie intraveineuse chez le rat (5 mg/kg d'indométacine) la suspension de nanocapsules conduit à un profil chronologique des concentrations plasmatiques d'indométacine mettant en évidence, par rapport à l'injection d'indométacine en solution, une distribution extravasculaire plus importante du principe actif (majoration d'un facteur voisin de 2 du volume de distribution de l'indométacine), suivie d'une élimination plus lente (majoration d'un facteur voisin de 2 de la demi-vie biologique de l'indométacine).

Example 3: Préparation de nanocapsules "vides"

On procède comme indiqué dans l'exemple 1, mais en remplaçant le benzoate de benzyle par de l'éther éthylique (0,5 ml). Après formation des nano-capsules, l'acétone et l'éther sont éliminés avec précaution sous pression réduite (trompe à eau), et la suspension de nanocapsules est concentrée par élimination de l'eau jusqu'au volume désiré, par exemple : 10 ml.

La taille des nanocapsules est de 228 nm avec un indice de disperison de 1,5.

Example 4: Préparation de nanocapsules de polymère contenant un colorant lipophile.

On procède comme indiqué dans l'exemple 3, mais en ajoutant 5 mg de Soudan III, colorant lipophile, au niveau de la phase acétonique.

Après élimination de l'acétone et de l'éther et concentration à 10 ml de la phase aqueuse dispersante, les nanocapsules ont une taille de 175 nm avec un indice de dispersion de 1.

La suspension de nanocapsules est ensuite mise en présence de 5 ml d'éther éthylique et l'ensemble est agité par retournement pendant 5 mn. La phase éthérée demeure incolore, ce qui démontre que le colorant lipophile est bien encapsulé.

Example 5 : Préparation de nanocapsules de polymère contenant un colorant hydrophile

D'une part, 125 mg d'acide poly (d,l) lactique, 3,5 mg d'éthythrosine, colorant hydrophile, et 0,4 ml de propylène-glycol, sont dissous dans 25 ml d'un mélange acétone/tétrahydrofuranne (50/50 v/v).

D'autre part, 0,8 ml de mono-oléate de sorbitanne (Span 80®), agent de surface non-ionique, est dissous dans 50 ml d'un mélange heptane/huile de silicone/acétone (80/10/10 v/v).

La phase acétone/tétrahydrofuranne est ajoutée, sous agitation magnétique, à la phase heptane/silicone/acétone. Après concentration au volume de 10 ml sous presion réduite, les nanocapsules ont une taille de 490 nm avec un indice de dispersion de 0,5.

La suspension de nanocapsules est, dans les mêmes conditions que dans l'exemple 4, agitée en présence de 5 ml d'eau purifiée. La phase aqueuse demeure incolore, ce qui démontre que le colorant hydrophile est bien encapsulé par le polymère.

Exemple 6: Préparation de nanocapsules contenant un lipide

On procède comme dans l'exemple 1, mais en remplaçant le benzoate de benzyle par une huile végétale (huile d'arachide, par exemple). Après filtration sur verre fritté, la suspension est additionnée d'un adjuvant de lyophilisation, le tréhalose (200 mg/ml), puis lyophilisée. Après remise en suspension de la poudre de nanocapsules dans 10 ml d'eau purifiée, la taille est de 490 nm avec un indice de dispersion de 2. Après repos prolongé, on n'observe aucune séparation de phase, ce qui démontre que l'huile est toujours encapsulée dans le polymère après lyophilisation.

Exemple 7 : Préparation de nanocapsules de polymère contenant un solide minéral D'une part, 125 mg d'un polymère acrylique (Eudragit L100, de la Société Röhm-Pharma) sont dissous dans 50 ml d'eau purifiée additionnée de 2 ml d'hydroxyde de sodium 0,1 N.

D'autre part, 125 mg de polymère mixte d'oxyde d'éthylène et de propylèneglycol (Pluronic F68$^R$) sont dissous dans 100 ml d'eau purifiée additionnée de 0,45 ml d'acide acétique pur, et 100 mg de carbure de silicium particulaire (taille moyenne 450 nm, indice de dispersion 1) sont dispersés au sein de cette phase.

On ajoute, sous agitation magnétique modérée (environ 100 r.p.m.) la phase polymérique alcaline à la dispersion de carbure de silicium dans la phase acide. Le polymère acrylique encapsule les particules solides de carbure de silicium, et les nanocapsules ainsi obtenues ont une taille moyenne de 475 nm avec un indice de dispersion de 1.

Les nanocapsules obtenues selon l'invention peuvent trouver des applications dans de nombreux secteurs techniques, notamment celles des microcapsules mais avec un gain important de taille (X10$^{-3}$), d'où une stabilité des suspensions et une utilisation plus facile.

En tant que "vecteurs" de médicaments, en thérapeutique humaine et animale les nanocapsules permettent d'envisager :
- d'atteindre de nouveaux sites d'action, en particulier intracellulaires, voire intralysosomiaux ;
- d'utiliser de nouvelles voies d'administration pour des médicaments connus, en augmentant la stabilité et/ou l'absorption des médicaments, ou en permettant la réalisation de formes injectables, par voie intravasculaire, de médicaments insolubles ;
- de modifier la distribution tissulaire des médicaments, par un meilleur ciblage vers les sites d'action favorables et/ou un détournement des sites d'effets indésirables voire toxiques (amélioration de l'index thérapeutique).

En pharmacie, les dispersions colloïdales de nanocapsules peuvent permettre :
- de réaliser des formes injectables de médicaments insolubles,
- de stabiliser un principe actif médicamenteux, et
- de réaliser des enrobages de formes galéniques à partir de dispersions aqueuses de polymères filmogènes.

Dans le domaine de l'agrochimie les nanocapsules peuvent véhiculer des insecticides, des pesticides. Leur taille peut permettre d'envisager une action plus puissante par une meilleure pénétration à travers la cuticule. La faible viscosité de la dispersion autorise une pulvérisation très facile sous forme de gouttelettes de très petite taille, plus efficaces car plus couvrantes.

Dans le domaine des peintures, vernis, et traitement des surfaces d'une manière générale, les nanocapsules permettent de véhiculer des pigments, des réactifs, des décapants. sous forme de dispersion aqueuse de très faible viscosité, aisés à pulvériser ou à appliquer, et qui peut, si nécessaire, être rendue visqueuse, voire adhésive (remise en suspension des nanocapsules dans un véhicule approprié). La taille réduite des nanocapsules conduit à une très grande finesse du dépôt et à une très grande homogénéité, par exemple de pigmentation.

Les nanocapsules obtenues selon l'invention peuvent aussi être utilisées dans les domaines de l'imprimerie et de la reprographie, du traitement de surface des textiles et fibres, de la photographie, de la lubrification.

**Revendications**

1. Procédé de préparation de systèmes colloïdaux dispersibles, sous forme de particules sphériques de type vésiculaires et de taille inférieure à 500 nm (nanocapsules), dont la paroi est constituée par une substance polymère, A et dont le noyau est constituée par une substance B, caractérisé en ce que :
   (1) on prépare une phase liquide constituée essentiellement par une solution de la substance polymère A dans un solvant ou dans un mélange de solvants, et contenant la substance B en solution ou en dispersion,
   (2) on prépare une seconde phase liquide constituée essentiellement par un non-solvant ou un mélange de non-solvants des substances A et B, et additionnée d' un ou de plusieurs surfactifs, le solvant ou le mélange de solvants de la première phase étant miscible en toutes proportions au non-solvant ou au melange de non-solvants de la seconde phase,
   (3) on ajoute, sous agitation modérée, la première phase à la seconde phase, de manière à obtenir une suspension colloïdale de nanocapsules,
   (4) si l'on désire, on élimine tout ou partie du solvant ou du mélange de solvants et de non-solvant ou du mélange de non-solvants, de manière à obtenir une suspension colloïdale de concentration voulue en nanocapsules ou à obtenir après addition de substances stabilisantes, une poudre de nanocapsules.

2. Procédé selon la revendication 1 caractérisé en ce que l'on ajoute dans l'étape (3) la phase liquide obtenue dans l'étape (1) à la phase liquide obtenue dans l'étape (2).

3. Procédé selon la revendication 1 ou la revendication 2, caractérisé en ce que le(s) surfactif(s) sont présents en une proportion de 0,1 à 10% en poids de la suspension colloïdale obtenue dans l'étape (3).

4. Procédé selon la revendication 3, caractérisé en ce que le(s) surfactif(s) sont présents en une proportion de 0,2 à 2% en poids.

5. Procédé selon la revendication 1, caractérisé en ce que la concentration de la substance polymère A, dans le solvant ou le mélange de solvants est comprise entre 0,1 et 10% en poids.

6. Procédé selon la revendication 5, caractérisé en ce que la concentration du polymère A est comprise entre 0,2 et 2% en poids.

7. Procédé selon l'une quelconque des revendications 1 à 6, caractérisé en ce que la substance B est une substance biologiquement active.

8. Procédé selon l'une quelconque des revendications 1 à 6, caractérisé en ce que la substance B est une huile.

9. Procédé selon la revendication 7 ou la revendication 8, caractérisé en ce que la substance B est une substance biologiquement active en solution dans une huile.

10. Procédé selon l'une quelconque des revendications 1 à 9, caractérisé en ce que dans l'étape (4) la totalité des solvants et des non-solvants est éliminée par lyophilisation.

11. Procédé selon l'une quelconque des revendications 1 à 10, caractérisé en ce que les nanocapsules ont une taille d'environ 150 à 450 nm.

**Claims**

1. Preparation process for dispersible colloidal systems, in the form of vesicular-type spherical particles and of a size smaller than 500 nm (nanocapsules), the wall of which is constituted by a polymer substance A and the nucleus of which is constituted by a substance B, characterized in that:
(1) a liquid phase is prepared constituted essentially by a solution of polymer substance A in a solvent or in a mixture of solvents, and containing substance B in solution or in dispersion,
(2) a second liquid phase is prepared constituted essentially by a non-solvent or a mixture of non-solvents of substances A and B, and having one or more surfactants added to it, the solvent or mixture of solvents of the first phase being miscible in all proportions with the non-solvent or with the mixture of non-solvents of the second phase,
(3) the first phase is added to the second phase under moderate agitation, so as to obtain a colloidal suspension of nanocapsules,
(4) if desired, all or part of the solvent or mixture of solvents and non-solvent or mixture of non-solvents are eliminated, so as to obtain a colloidal suspension of desired nanocapsule concentration or so as to obtain, after the addition of stabilizing substances, a powder of nanocapsules.

2. Process according to claim 1, characterized in that in stage (3) the liquid phase obtained in stage (1) is added to the liquid phase obtained in stage (2).

3. Process according to claim 1 or claim 2, characterized in that the surfactant(s) is(are) present in a proportion of 0.1 to 10% by weight of the colloidal suspension obtained in stage (3).

4. Process according to claim 3, characterized in that the surfactant(s) is (are) present in a proportion of 0.2 to 2% by weight.

5. Process according to claim 1, characterized in that the concentration of the polymer substance A in the solvent or mixture of solvents is comprised between 0.1 and 10% by weight.

6. Process according to claim 5, characterized in that the concentration of polymer A is comprised between 0.2 and 2% by weight.

7. Process according to any one of claims 1 to 6, characterized in that substance B is a biologically active substance.

8. Process according to any one of claims 1 to 6, characterized in that substance B is an oil.

9. Process according to claim 7 or claim 8, characterized in that substance B is a biologically active substance in solution in an oil.

10. Process according to any one of claims 1 to 9, characterized in that in stage (4) all of the solvents and non-solvents are eliminated by lyophilization.

11. Process according to any one of claims 1 to 10, characterized in that the nanaocapsules are of a size of about 150 to 450 nm.

**Patentansprüche**

1. Verfahren zur Herstellung kolloid-disperser Systeme in Form kugelförmiger, bläschenartiger Teilchen von unter 500 nm Größe (Nanokapseln), deren Wand aus einer polymeren Substanz A besteht und deren Kern aus einer Substanz B besteht, dadurch gekennzeichnet, daß

(1) man eine flüssige Phase herstellt, welche im wesentlichen aus einer Lösung der polymeren Substanz A in einem Lösungsmittel oder einem Lösungsmittelgemisch besteht und welche die Substanz B in Lösung oder als Dispersion enthält,

(2) man eine zweite flüssige Phase herstellt, welche im wesentlichen aus einem Lösungsmittel oder einem Lösungsmittelgemisch, welche die Substanzen A und B nicht lösen, besteht, und welcher ein oder mehrere oberflächenaktive Stoffe zugesetzt sind, wobei das Lösungsmittel oder das Lösungsmittelgemisch der ersten Phase in allen Verhältnissen mit dem Lösungsmittel oder dem Lösungsmittelgemisch der zweiten Phase, welche die Substanz nicht lösen, mischbar sind,

(3) man unter mäßigem Rühren die erste Phase der zweiten Phase zufügt, um eine kolloidale Suspension von Nanokapseln zu erhalten, und

(4) falls gewünscht, man das gesamte oder einen Teil des Lösungsmittels oder Lösungsmittelgemisches und des Lösungsmittels oder des Lösungsmittelgemisches, welche die Substanzen nicht lösen, entfernt, um eine kolloidale Suspension der gewünschten Konzentration an Nanokapseln zu erhalten oder um nach Zugabe von Stabilisatoren ein Nanokapsel-Pulver zu erhalten.

2. Verfahren gemäß Anspruch 1, dadurch gekennzeichnet, daß man in Schritt (3) die in Schritt (1) erhaltene flüssige Phase der in Schritt (2) erhaltenen flüssigen Phase zufügt.

3. Verfahren gemäß Anspruch 1 oder Anspruch 2, dadurch gekennzeichnet, daß das (die) oberflächenaktive(n) Mittel in einem Verhältnis von 0,1 bis 10 Gew.-% bezogen auf die in Schritt (3) erhaltene kolloidale Suspension vorhanden sind.

4. Verfahren gemäß Anspruch 3, dadurch gekennzeichnet, daß das (die) oberflächenaktive-(n) Mittel in einem Verhältnis von 0,2 bis 2 Gew.-% vorhanden sind.

5. Verfahren gemäß Anspruch 1, dadurch gekennzeichnet, daß die Konzentration der polymeren Substanz A im Lösungsmittel oder dem Lösungsmittelgemisch zwischen 0,1 und 10 Gew.-% liegt.

6. Verfahren gemäß Anspruch 5, dadurch gekennzeichnet, daß die Konzentration des Polymers A zwischen 0,2 und 2 Gew.-% liegt.

7. Verfahren gemäß einem der Ansprüche 1 bis 6, dadurch gekennzeichnet, daß die Substanz B eine biologisch aktive Substanz ist.

8. Verfahren gemäß einem der Ansprüche 1 bis 6, dadurch gekennzeichnet, daß die Substanz B ein Öl ist.

9. Verfahren gemäß Anspruch 7 oder Anspruch 8, dadurch gekennzeichnet, daß die Substanz B eine in einem Öl gelöste biologisch aktive Substanz ist.

10. Verfahren gemäß einem der Ansprüche 1 bis 9, dadurch gekennzeichnet, daß in Schritt (4) die gesamten Lösungsmittel und nichtlösenden Lösungsmittel durch Gefriertrocknung entfernt werden.

11. Verfahren gemäß einem der Ansprüche 1 bis 10, dadurch gekennzeichnet, daß die Nanokapseln eine Größe von etwa 150 bis 450 nm haben.